# EUROPÄISCHE PATENTSCHRIFT

(11) **EP 2 579 912 B1**
(45) Veröffentlichungstag und Bekanntmachung des Hinweises auf die Patenterteilung: **04.01.2017**
(21) Anmeldenummer: 11732366.7
(22) Anmeldetag: 14.06.2011
(51) Int. Cl.: A61M 1/16

(54) **VORRICHTUNG UND VERFAHREN ZUM FÖRDERN VON FLÜSSIGKEITEN IN DIE BEHANDLUNGSEINHEIT EINER MEDIZINISCHEN BEHANDLUNGSVORRICHTUNG, INSBESONDERE IN DEN DIALYSATOR EINER DIALYSEVORRICHTUNG**
METHOD AND DEVICE FOR DELIVERING LIQUIDS INTO THE TREATMENT UNIT OF A MEDICAL TREATMENT DEVICE, IN PARTICULAR INTO THE DIALYSER OF A DIALYSIS DEVICE
DISPOSITIF ET PROCÉDÉ DE CIRCULATION DE LIQUIDES DANS L'UNITÉ DE TRAITEMENT D'UN DISPOSITIF DE TRAITEMENT MÉDICAL, EN PARTICULIER DANS LE DIALYSEUR D'UN DISPOSITIF DE DIALYSE

(30) Priorität: 14.06.2010 DE 102010023635
(43) Veröffentlichungstag der Anmeldung: 17.04.2013
(73) Patentinhaber: Fresenius Medical Care Deutschland GmbH, 61352 Bad Homburg v.d.H. (DE)
(72) Erfinder: BRANDL, Matthias, 97631 Bad Königshofen (DE); WEIS, Manfred, 66606 St. Wendel (DE); NIER, Volker, 61203 Reichelsheim (DE); MAGER, Gerhard, 61352 Bad Homburg (DE); HERRENBAUER, Michael, 61267 Neu-Anspach (DE)
(74) Vertreter: Oppermann, Frank
(86) Internationale Anmeldenummer: PCT/EP2011/002915
(87) Internationale Veröffentlichungsnummer: WO 2011/157396

(56) Entgegenhaltungen:
- EP-A1- 2 005 982
- DE-A1- 4 239 937
- DE-A1- 19 702 211
- DE-T2- 69 400 279
- DE-U1- 29 902 953
- US-A- 4 209 391
- US-A1- 2009 198 170

## Beschreibung

Die Erfindung betrifft eine Vorrichtung zum Fördern von Flüssigkeiten in die Behandlungseinheit einer medizinischen Behandlungsvorrichtung, insbesondere in den Dialysator einer Dialysevorrichtung. Darüber hinaus betrifft die Erfindung eine extrakorporale Blutbehandlungsvorrichtung, insbesondere Dialysevorrichtung, die über eine Vorrichtung zum Fördern von Flüssigkeiten in die Behandlungseinheit, insbesondere den Dialysator, der Blutbehandlungsvorrichtung, insbesondere Dialysevorrichtung, verfügt.

Es sind verschiedene Arten von Behandlungsvorrichtungen bekannt, die über eine mit einer Flüssigkeit zu versorgende Behandlungseinheit verfügen. Zu den bekannten Behandlungsvorrichtungen gehören beispielsweise die Blutbehandlungsvorrichtungen. Während der Blutbehandlung strömt das Blut des Patienten in einem extrakorporalen Blutkreislauf durch die Blutbehandlungseinheit. Bei den Vorrichtungen zur Hämodialyse, Hämofiltration und Hämodiafiltration ist die Blutbehandlungseinheit ein Dialysator oder Filter, der durch eine semipermeable Membran in eine Blutkammer und eine Dialysierflüssigkeitskammer getrennt ist. Während der Dialysebehandlung strömt das Blut in einem extrakorporalen Blutkreislauf durch die Blutkammer, während die Dialysierflüssigkeit in einem Dialysierflüssigkeitskreislauf durch die Dialysierflüssigkeitskammer des Dialysators strömt.

Wegen der großen Austauschmengen besteht bei den bekannten Verfahren und Vorrichtungen zur Blutbehandlung die Notwendigkeit einer exakten Bilanzierung der dem Patienten entzogenen Flüssigkeit und der dem Patienten zugeführten Flüssigkeit über die gesamte Behandlungszeit. Zum Stand der Technik gehören gravimetrische und volumetrische Bilanziervorrichtungen.

Eine Hämodiafiltrationsvorrichtung mit volumetrischer Bilanzierung ist beispielsweise aus der DE 26 34 238 A1 bekannt. Die Bilanziervorrichtung der bekannten Hämodiafiltrationsvorrichtung weist einen volumenstarren Hohlkörper auf, der durch eine bewegliche Trennwand in zwei Kammern unterteilt ist. Jede Kammer weist einen Einlass und einen Auslass auf, an denen Zuführ- und Abführleitungen für frische bzw. verbrauchte Dialysierflüssigkeit angeordnet sind, wobei in jede Leitung ein Absperrorgan geschaltet ist. Darüber hinaus sind Pumpen für die Förderung der frischen und verbrauchten Dialysierflüssigkeit sowie eine Steuereinheit vorgesehen, die ein wechselseitiges Befüllen der beiden Kammern erlaubt.

Um einen kontinuierlichen Fluss von Dialysierflüssigkeit durch die Dialysierflüssigkeitskammer des Dialysators sicherstellen zu können, werden in der Praxis zwei Bilanzkammern parallel geschaltet, die den Dialysator wechselseitig mit frischer Dialysierflüssigkeit versorgen. Eine Bilanziereinheit mit zwei Bilanzkammern ist beispielsweise aus der DE 28 38 414 bekannt.

Während einer Dialysebehandlung beträgt der Dialysierflüssigkeitsfluss typischerweise 500 ml/min, kann aber in Abhängigkeit von der jeweiligen Behandlungssituation bis zu 1000 ml/min betragen. Bei einer Dialysedauer von 4 Stunden bedeutet dies einen Dialysierflüssigkeitsbedarf, der typischerweise zwischen 120 1 beträgt, in Abhängigkeit von der jeweiligen Behandlungssituation aber auch über 200 1 liegen kann.

Aufgrund des großen Flüssigkeitsbedarfs bei der Dialyse hat sich die Herstellung des Dialysats aus Konzentraten und Reinwasser (RO-Wasser) in der Maschine etabliert, um die Vorhaltung größerer Mengen an Lösungen zu vermeiden. Das RO-Wasser wird zentral in der Klinik bereitgestellt und an die Dialaysemaschine in den Dialysestationen über Leitungen verteilt.

Bei der Behandlung einer akuten Niereninsuffizienz, wie sie beispielsweise nach Unfällen vorkommen kann, die eine intensivmedizinische Betreuung des Patienten notwendig macht, ist im allgemeinen ein RO-Wasseranschluss nicht vorhanden. Die Dialysierflüssigkeit wird der Maschine dann über Behältnisse, beispielsweise Kanister oder Beutel, zur Verfügung gestellt.

Um den Handhabungsaufwand möglichst gering zu halten, wird insbesondere bei der intensivmedizinischen Betreuung einer akuten Niereninsuffizienz versucht, den Bedarf an Dialysierflüssigkeit zu verringern. Dies gelingt dadurch, dass die Dialysierflüssigkeit für eine gewisse Zeit über den Dialysator rezirkuliert wird. Damit kann der Dialysatbedarf auf Werte reduziert werden, die unter 100 ml/min liegen.

Eine Blutbehandlungsvorrichtung mit einem Rezirkulationskreislauf ist beispielsweise aus der US 5 685 988 bekannt. Die Rezirkulation von Dialysierflüssigkeit soll aber nur der Bestimmung von Blutbehandlungsparametern dienen.

Aus der US 2009/0198170 A1 ist eine Dialysevorrichtung bekannt, die über eine Bilanziereinheit mit zwei Bilanzkammern verfügt, die jeweils in zwei Kammern unterteilt sind. Zu- und Abläufe der Bilanzkammern können mit Ventilen verschlossen werden. Frische Dialysierflüssigkeit fließt über eine Leitung aus der Bilanziereinheit in einen Dialysator und strömt aus dem Dialysator über die Leitung zurück zu der Bilanziereinheit. Die frische Dialysierflüssigkeit wird in Beuteln bereitgestellt und die verbrauchte Dialysierflüssigkeit fließt in einen Abfluss. Die bekannte Dialysevorrichtung sieht als Bypassleitung einen Leitungspfad vor, der durch einen Filter führt. Während Dialysierflüssigkeit von der Bilanziereinheit zu dem Dialysator und von dem Dialysator zu der Bilanziereinheit strömt, wird ein Teil der Dialysierflüssigkeit mit einer Pumpe mehrfach durch den Filter gepumpt.

Aus der DE 197 02 211 A1 ist eine Vorrichtung zur Steuerung des Flüssigkeitsentzugs bei der Hämodialyse bekannt, mit der die zum Dialysator führende Zulaufleitung mit der vom Dialysator abgehenden Ablaufleitung verbunden wird. Im Rezirkulationskreislauf verfügt die Vorrichtung nur über eine Pumpe, die in der Ablaufleitung angeordnet ist. Daher kann auch die dem Fachmann keinen Hinweis für die Anordnung von zwei Pumpen in der Zulaufleitung geben.

Die DE 299 02 953 schlägt vor, einen im Dialysierflüssigkeitskreislauf angeordneten Filter über eine Bypassleitung zu spülen.

Die DE 42 39 937 A1 beschreibt ein Verfahren zur Überprüfung der Funktionsfähigkeit einer Teileinrichtung eines Hamodialysegerätes, wobei im Dialysierflüssigkeitssystem eine den Dialysator umgehende Bypassleitung vorgesehen ist. Zum Fördern der Dialysierflüssigkeit ist im Dialysierflüssigkeitssystem nur eine Pumpe vorgesehen.

Die EP 2 005 982 A1 beschreibt eine Dialysevorrichtung, die über eine Messeinheit verfügt. Zur Einbindung der Messeinheit in den Dialysierflüssigkeitsfluss weist die Dialysevorrichtung einen Rezirkulationskreislauf auf, der eine Zu- und Ablaufleitung umfasst, in der Ventile angeordnet sind, um den Zu- bzw. Abfluss in die Messeinheit zu unterbrechen. Die bekannte Dialysevorrichtung sieht die Rezirkulation von Dialysierflüssigkeit nur zu Messzwecken vor.

Aus der US 4 209 391 A ist eine Vorrichtung zur Regelung der Menge der dem Patienten entzogenen Flüssigkeit bekannt, bei der im Dialysierflüssigkeitskreislauf zwischen Bilanziereinheit und Dialysator ebenfalls nur eine Dialysierflüssigkeitspumpe vorgesehen ist.

Der Erfindung liegt die Aufgabe zugrunde, eine Vorrichtung zum Fördern von Flüssigkeiten in die Behandlungseinheit einer medizinischen Behandlungsvorrichtung, insbesondere in den Dialysator einer Dialysevorrichtung, zu schaffen, mit der sich der Bedarf an Dialysierflüssigkeit verringern lässt.

Die Lösung dieser Aufgabe erfolgt erfindungsgemäß mit den Merkmalen der unabhängigen Patentansprüche. Vorteilhafte Ausführungsformen der Erfindung sind Gegenstand der Unteransprüche.

Die erfindungsgemäße Vorrichtung beruht darauf, dass die Flüssigkeit, mit der die Behandlungseinheit versorgt wird, in einem die Behandlungseinheit einschließenden Flüssigkeitskreislauf zirkuliert. Zur Bilanzierung von frischer und verbrauchter Flüssigkeit, die der Behandlungseinheit zugeführt oder aus der Behandlungseinheit abgeführt wird, dient eine Bilanziereinheit, die grundsätzlich eine oder zwei Bilanzkammern aufweisen kann.

Die erfindungsgemäße Vorrichtung zeichnet sich dadurch aus, dass die Bilanzkammer der Bilanziereinheit, oder die beiden Bilanzkammern der Bilanziereinheit in den die Behandlungseinheit einschließenden Flüssigkeitskreislauf eingebunden werden kann. Dadurch ist es möglich, dem Flüssigkeitskreislauf kontinuierlich frische Flüssigkeit zuzuführen bzw. verbrauchte Flüssigkeit aus dem Flüssigkeitskreislauf abzuführen. Dabei kann die Zu- und Abfuhr von frischer bzw. verbrauchter Flüssigkeit mit einer anderen Flussrate erfolgen, als mit der Flussrate, mit der die Flüssigkeit in dem Flüssigkeitskreislauf über die Behandlungseinheit zirkuliert. Folglich stellt sich in dem Flüssigkeitskreislauf eine "Flüssigkeit" ein, die in Abhängigkeit von dem Verhältnis der Flussraten in der Konzentration zwischen einer "frischen Flüssigkeit" und einer "verbrauchten Flüssigkeit" liegt. Dem die Blutbehandlungseinheit, insbesondere den Dialysator, einschließenden Flüssigkeitskreislauf kann unabhängig von der Zufuhr bzw. Abfuhr von frischer bzw. verbrauchter Flüssigkeit auch Flüssigkeit (Ultrafiltrat) entzogen werden.

Die Mittel zum Fördern von Flüssigkeit weisen zwei Pumpen auf, die in der von der Bilanzkammer zu der Blutbehandlungseinheit führenden Abflussleitung angeordnet sind. Von diesen beiden Pumpen ist die eine Pumpe in dem Abschnitt dieser Abflussleitung angeordnet, der zu dem Punkt führt, an dem der eine Anschluss des Bypasses an die Abflussleitung angeschlossen ist, während die andere Pumpe in dem Abschnitt dieser Abflussleitung angeordnet ist, der von dem Anschlusspunkt des Bypasses abgeht. Die Flussraten dieser beiden Pumpen in der Abflussleitung geben die Flussrate vor, mit der frische Flüssigkeit dem Flüssigkeitskreislauf zugeführt bzw. verbrauchte Flüssigkeit aus dem Flüssigkeitskreislauf abgeführt wird.

Bei einer bevorzugten Ausführungsform der Erfindung ist die Flussrate, mit der die Flüssigkeit über die Behandlungseinheit in dem Flüssigkeitskreislauf zirkuliert, größer als die Flussrate, mit der Flüssigkeit dem Flüssigkeitskreislauf zu- bzw. abgeführt wird.

Die erfindungsgemäße Vorrichtung verfügt über einen Bypass, der die von der Bilanzkammer zu der Behandlungseinheit führende Abflussleitung mit der von der Behandlungseinheit zu der Bilanzkammer führenden Zuflussleitung verbindet. Der Bypass erlaubt nicht nur eine kontinuierliche Zufuhr frischer Flüssigkeit in den die Behandlungseinheit einschließenden Flüssigkeitskreislauf, sondern auch die Aufrechterhaltung eines Flüssigkeitsflusses durch die Blutbehandlungseinheit, wenn die Bilanzkammer der Bilanziereinheit mit frischer Flüssigkeit unter Verwerfung verbrauchter Flüssigkeit befüllt wird. Wenn eine Bilanziereinheit mit zwei wechselseitig arbeitenden Bilanzkammern verwendet wird, kommt dieser Vorteil allerdings nicht zum Tragen. Eine besonders bevorzugte Ausführungsform der Erfindung sieht daher eine Bilanziereinheit mit nur einer Bilanzkammer vor. Bei dieser besonders bevorzugten Ausführungsform stellt der Bypass sicher, dass beim Umschalten der Bilanzkammer die Flüssigkeitsströmung durch die Blutbehandlungseinheit nicht abbricht. Dadurch ergibt sich ein vereinfachter Aufbau der Bilanziereinheit.

Die Mittel zum Fördern von Flüssigkeit in die bzw. aus der Bilanzkammer und die Mittel zum Unterbrechen des Zuflusses von Flüssigkeit in die Bilanzkammer bzw. des Abflusses von Flüssigkeit aus der Bilanzkammer können unterschiedlich ausgebildet sein. Zum Fördern von Flüssigkeit dienen vorzugsweise die bekannten okkludierenden Pumpen, in die sich Schlauchleitungen einlegen lassen. Zum Unterbrechen des Zuflusses bzw. Abflusses von Flüssigkeit dienen vorzugsweise die bekannten elektromagnetisch oder pneumatisch betätigbaren Absperrorgane, die in den Leitungen angeordnet sind. Eine Steuereinheit steuert die Mittel zum Fördern von Flüssigkeit und die Mittel zum Unterbrechen des Zuflusses bzw. Abflusses von Flüssigkeit an. Da okkludierende Pumpen im Stillstand die Schlauchleitung abklemmen, können die okkludierenden Pumpen auch Absperrorgane ersetzen.

Bei einer besonders bevorzugten Ausführungsform weisen die Mittel zum Fördern von Flüssigkeit eine weitere Pumpe auf, die in der von der Flüssigkeitsquelle zu der Bilanzkammer führenden Zuflussleitung angeordnet ist.

Bei der besonders bevorzugten Ausführungsform umfassen die Mittel zum Unterbrechen des Zuflusses und/oder Abflusses von Flüssigkeit ein erstes Absperrorgan, das in der von der Flüssigkeitsquelle zu der Bilanzkammer führenden ersten Zuflussleitung angeordnet ist, ein zweites Absperrorgan, das in der von der Bilanzkammer abgehenden und zu dem Abfluss führenden zweiten Abflussleitung angeordnet ist, ein drittes Absperrorgan, das in der von der Bilanzkammer abgehenden und zu der Blutbehandlungseinheit führenden zweiten Abflussleitung angeordnet ist, und ein viertes Absperrorgan, das in der von der Behandlungseinheit zu der Bilanzkammer führenden zweiten Zuflussleitung angeordnet ist. Sämtliche Absperrorgane werden von der Steuereinheit angesteuert.

Die Steuereinheit ist bei der besonders bevorzugten Ausführungsform derart ausgebildet, dass in einem ersten Arbeitstakt eines ersten Arbeitszyklus von aufeinander folgenden Arbeitszyklen das erste und zweite Absperrorgan geöffnet und das dritte und vierte Absperrorgan geschlossen sind, wobei die erste und dritte Pumpe in Betrieb sind. In dem ersten Arbeitstakt wird die Bilanzkammer mit frischer Flüssigkeit unter Verwerfung verbrauchter Flüssigkeit befüllt. Während des Füllvorgangs der Bilanzkammer ist die Flüssigkeitsströmung durch die Behandlungseinheit nicht unterbrochen. An den ersten Arbeitstakt schließt sich ein zweiter Arbeitstakt an, in dem das erste und zweite Absperrorgan geschlossen und das dritte und vierte Absperrorgan geöffnet sind, wobei die zweite und dritte Pumpe in Betrieb sind. In dem zweiten Arbeitstakt zirkuliert die Flüssigkeit in dem die Behandlungseinheit einschließenden Flüssigkeitskreislauf. Dabei kann, muss aber nicht, frische Flüssigkeit dem Flüssigkeitskreislauf zugeführt bzw. aus dem Flüssigkeitskreislauf abgeführt werden.

Eine weitere bevorzugte Ausführungsform sieht die Integration eines weiteren Absperrorgans im Bypass vor. Dieses Absperrorgan dient der besseren Befüllung und Entlüftung des Systems vor der Durchführung der Behandlung. Andererseits kann mit dem Absperrorgan im Bypass auch die Zirkulation in dem Flüssigkeitskreislauf unterbrochen werden.

Im Folgenden werden Ausführungsbeispiele der Erfindung unter Bezugnahme auf die Zeichnungen näher erläutert.

Es zeigen:
- Fig. 1: Eine Hämodialysevorrichtung mit einer Vorrichtung zur Versorgung des Dialysators der Dialysevorrichtung mit Dialysierflüssigkeit in stark vereinfachter schematischer Darstellung, wobei der erste Arbeitstakt eines Arbeitszyklus dargestellt ist,
- Fig. 2: die Hämodialysevorrichtung von Fig. 2, wobei der zweite Arbeitstakt des Arbeitszyklus dargestellt ist,
- Fig. 3: ein zweites Ausführungsbeispiel der Hämodialysevorrichtung mit der Vorrichtung zur Versorgung des Dialysators mit Dialysierflüssigkeit in stark vereinfachter schematischer Darstellung,
- Fig. 4: eine alternative Ausführungsform der Hämodialysevorrichtung von Fig. 3,
- Fig. 5: ein weiteres Ausführungsbeispiel der Dialysevorrichtung in stark vereinfachter schematischer Darstellung und
- Fig. 6: eine alternative Ausführungsform der Dialysevorrichtung von Fig. 5.

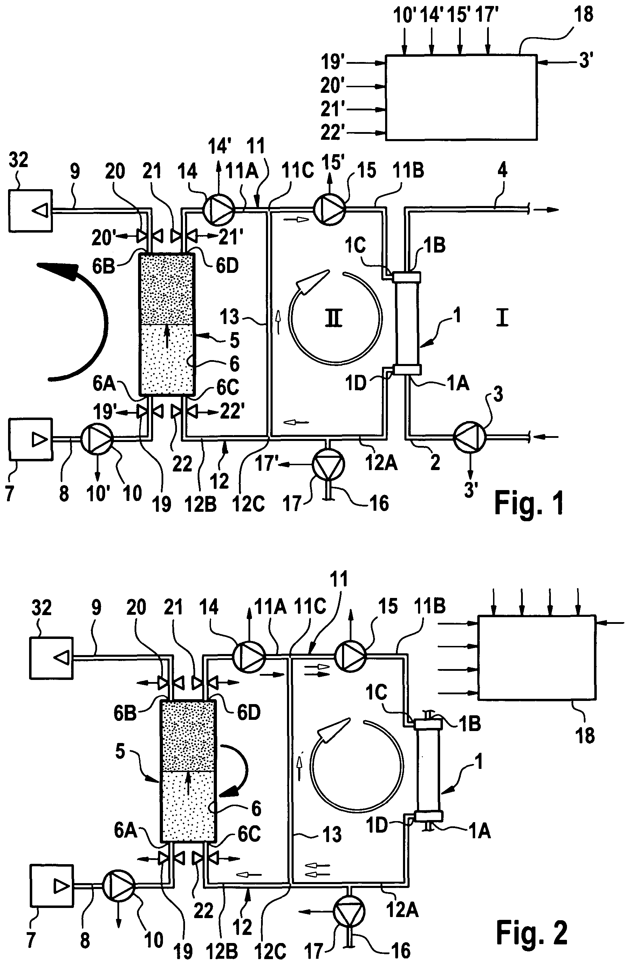

Die Erfindung wird nachfolgend am Beispiel einer Blutbehandlungsvorrichtung beschrieben, die über einen Dialysator als Blutbehandlungseinheit verfügt. Fig.1 zeigt in stark vereinfachter schematischer Darstellung die wesentlichen Komponenten der Hämodialysevorrichtung. Die Vorrichtung zur Versorgung des Dialysators mit Dialysierflüssigkeit ist Bestandteil der Dialysevorrichtung.

Die Dialysevorrichtung weist einen Dialysator 1 auf, der durch eine nicht dargestellte semipermeable Membran in eine nicht dargestellte Blutkammer und Dialysierflüssigkeitskammer unterteilt ist. Von dem Patienten führt eine Blutzuführleitung 2, in die eine Blutpumpe 3 geschaltet ist, zu einem Einlass 1A der Blutkammer des Dialysators 1, während von einem Auslass 1B der Blutkammer des Dialysators 1 eine Blutabführleitung 4 abgeht, die zu dem Patienten führt. Während der Blutbehandlung strömt das Blut des Patienten in dem extrakorporalen Blutkreislauf I durch die Blutkammer des Dialysators 1.

Der Dialysator 1 wird mit Dialysierflüssigkeit versorgt, die durch die Dialysierflüssigkeitskammer des Dialysators 1 strömt. Nachfolgend wird die Vorrichtung zum Fördern der Dialysierflüssigkeit in den Dialysator 1 beschrieben.

Zum Bilanzieren von frischer gegen verbrauchte Dialysierflüssigkeit dient eine Bilanziereinheit 5, die bei dem vorliegenden Ausführungsbeispiel nur über eine Bilanzkammer 6 verfügt. Die Bilanzkammer 6 weist an der Unterseite einen ersten Einlass 6A und an der Oberseite einen ersten Auslass 6B sowie an der Unterseite einen zweiten Einlass 6C und an der Oberseite einen zweiten Auslass 6D auf.

Die Dialysierflüssigkeit wird in einer Dialysierflüssigkeitsquelle 7 bereitgestellt, bei der es sich um einen Kanister oder einen Beutel handeln kann. Von der Dialysierflüssigkeitsquelle 7 geht eine erste Zuflussleitung 8 ab, die zu dem ersten Einlass 6A der Bilanzkammer 6 führt. Von dem ersten Auslass 6B der Bilanzkammer 6 geht eine erste Abflussleitung 9 ab, die zu einem Ablauf oder Abfluss 32 führt. In die erste Zuflussleitung 8 ist eine Blutpumpe 10, insbesondere eine okkludierende Pumpe, geschaltet, die frische Dialysierflüssigkeit aus der Dialysierflüssigkeitsquelle 7 in die Bilanzkammer 6 fördert.

Von dem zweiten Auslass 6D der Bilanzkammer 6 geht eine zweite Abflussleitung 11 ab, die zu dem Einlass 1C der Dialysierflüssigkeitskammer des Dialysators 1 führt. Von dem Auslass 1 D der Dialysierflüssigkeitskammer des Dialysators 1 geht eine zweite Zuflussleitung 12 ab, die zu dem zweiten Einlass 6C der Bilanzkammer 6 führt.

Bei den Zu- und Abflussleitungen 8, 9, 11, 12 handelt es sich um Schlauchleitungen. Die zweite Abflussleitung 11 weist in Strömungsrichtung einen ersten Abschnitt 11A und einen zweiten Abschnitt 11 B auf, während die zweite Zuflussleitung 12 in Strömungsrichtung einen ersten Abschnitt 12A und einen zweiten Abschnitt 12B aufweist.

Die zweite Abflussleitung 11 und die zweite Zuflussleitung 12 sind über einen Bypass 13 verbunden. Bei dem Bypass 13 handelt es sich um eine Leitung, die mit dem einen Ende an dem Verbindungspunkt 11C zwischen dem ersten Abschnitt 11A und dem zweiten Abschnitt 11 B der zweiten Abflussleitung 11 und mit dem anderen Ende an dem Verbindungspunkt 12C zwischen dem ersten Abschnitt 12A und dem zweiten Abschnitt 12B der zweiten Zuflussleitung 12 angeschlossen ist. Mit dem Bypass 13 wird ein Flüssigkeitskreislauf II geschaffen, der die Dialysierflüssigkeitskammer des Dialysators 1 einschließt. Der Flüssigkeitskreislauf II umfasst die Bypass-Leitung 13, den zweiten Abschnitt 11B der zweiten Abflussleitung 11, die Dialysierflüssigkeitskammer des Dialysators 1 und den ersten Abschnitt 12A der zweiten Zuflussleitung 12.

In den ersten Abschnitt 11 A der zweiten Abflussleitung 11 ist eine zweite Pumpe 14 und in den zweiten Abschnitt 11B der zweiten Abflussleitung 11 eine dritte Pumpe 15 geschaltet. Von dem ersten Abschnitt 12A der zweiten Zuflussleitung 12 geht eine Ultrafiltratleitung 16 ab, in die eine vierte Pumpe 17 geschaltet ist, mit der dem Flüssigkeitskreislauf II Flüssigkeit (Ultrafiltrat) entzogen werden kann. Die vier Pumpen 10, 14, 15, 17 sind über Steuerleitungen 10', 14', 15', 17' mit einer Steuereinheit 18 verbunden. Die Steuereinheit 18 ist bei dem vorliegenden Beispiel Bestandteil der zentralen Steuereinheit der Dialysevorrichtung. Die zentrale Steuereinheit 18 der Dialysevorrichtung ist über eine Steuerleitung 3' auch mit der Blutpumpe 3 verbunden.

In die erste Zuflussleitung 8 ist zwischen der ersten Pumpe 10 und der Bilanzkammer 6 ein erstes Absperrorgan 19 geschaltet, während in die erste Abflussleitung 9 ein zweites Absperrorgan 20 geschaltet ist. In die zweite Abflussleitung 11 ist zwischen der Bilanzkammer 6 und der zweiten Pumpe 14 ein drittes Absperrorgan 21 geschaltet, während in die zweite Zuflussleitung 12 zwischen dem Verbindungspunkt 12C und der Bilanzierkammer 6 ein drittes Absperrorgan 22 geschaltet ist. Die Absperrorgane 19, 20, 21, 22 sind elektromagnetisch betätigbare Schlauchklemmen, die über Steuerleitungen 19', 20', 21', 22' mit der zentralen Steuereinheit 18 verbunden sind.

Die zentrale Steuereinheit 18 steuert die Pumpen 10, 14, 15 wie folgt an. Die Dialysevorrichtung wird in aufeinanderfolgenden Zyklen betrieben, die jeweils zwei Arbeitstakte umfassen. Fig. 2 zeigt den ersten Arbeitstakt und Fig. 2 den zweiten Arbeitstakt eines Arbeitszyklus.

Der erste Arbeitstakt umfasst das Befüllen der Bilanzierkammer 6, während Dialysierflüssigkeit durch den Dialysator 1 strömt. Die zentrale Steuereinheit 18 öffnet das erste und zweite Absperrorgan 19, 20 und schließt das dritte und vierte Absperrorgan 21, 22. Dabei setzt die Steuereinheit 18 die erste Pumpe 10 und die dritte Pumpe 15 in Betrieb. Die zweite Pumpe 14 steht still. Da die zweite okkludierende Pumpe 14 stillsteht, kann das dritte Absperrorgan 21 auch offen sein.

Die erste Pumpe 10 fördert frische Dialysierflüssigkeit aus der Dialysierflüssigkeitsquelle 7 in die Bilanzierkammer 6, die in dem zweiten Arbeitstakt des vorausgehenden Arbeitszyklus mit verbrauchter Dialysierflüssigkeit befüllt worden ist. Während sich die Bilanzkammer 6 mit frischer Dialysierflüssigkeit füllt, wird die verbrauchte Dialysierflüssigkeit über die erste Abflussleitung 9 in den Abfluss 32 verworfen. Die erste Pumpe 10 läuft solange, bis in der Bilanzkammer 6 verbrauchte Dialysierflüssigkeit vollständig gegen frische Dialysieflüssigkeit ausgetauscht ist. Während des Befüllens der Bilanzkammer 6 mit frischer Dialysierflüssigkeit ist die Flüssigkeitsströmung durch den Dialysator 1 nicht unterbrochen. Die dritte Pumpe 15 fördert die Dialysierflüssigkeit in dem Flüssigkeitskreislauf II, der den zweiten Abschnitt 11 B der zweiten Abflussleitung 11, den Dialysator 1, den ersten Abschnitt 12A der zweiten Zuflussleitung 12 und die Bypass-Leitung 13 einschließt. Die Bilanzkammer 6 sollte möglichst schnell mit frischer Dialysierflüssigkeit befüllt werden, so dass die Dialysierflüssigkeit nur eine kurze Zeitdauer in dem Flüssigkeitskreislauf zirkuliert (Fig. 1A).

An den ersten Arbeitstakt (Fig. 1) schließt sich der zweite Arbeitstakt (Fig. 2) an. In dem zweiten Arbeitstakt schließt die zentrale Steuereinheit 18 das erste und zweite Absperrorgan 19, 20 und öffnet das dritte und vierte Absperrorgan 21, 22. Weiterhin hält die Steuereinheit 18 die erste Pumpe 10 an und setzt die zweite Pumpe 14 in Betrieb. Folglich laufen die zweite und dritte Pumpe 14, 15. Die Steuereinheit 18 gibt für die zweite Pumpe 14 eine kleinere Förderrate vor als für die dritte Pumpe 15. Folglich strömt in dem Flüssigkeitskreislauf II Dialysierflüssigkeit mit einer Flussrate, die der Differenz von den Förderraten der dritten und zweiten Pumpe 15, 14 entspricht. Diese Förderrate QD_{fast} kann relativ hoch sein.

Während Dialysierflüssigkeit in dem Flüssigkeitskreislauf II durch den Dialysator 1 zirkuliert, wird dem Flüssigkeitskreislauf II ständig frische Dialysierflüssigkeit zugeführt und verbrauchte Dialysierflüssigkeit dem Flüssigkeitskreislauf 12 entzogen. Die frische Dialysierflüssigkeit wird mit der von der zweiten Pumpe 14 vorgegebenen Förderrate über den ersten Abschnitt 11A der zweiten Abflussleitung 11, die an den zweiten Auslass 6D der Bilanzierkammer 6 angeschlossen ist, dem Flüssigkeitskreislauf II zugeführt. Über den zweiten Abschnitt 12B der zweiten Zuflussleitung 12, die an dem zweiten Einlass 6C der Bilanzkammer 6 angeschlossen ist, wird verbrauchte Dialysierflüssigkeit dem Flüssigkeitskreislauf II entzogen. Die Dialysevorrichtung erlaubt auch dem Flüssigkeitskreislauf II Flüssigkeit (Ultrafiltrat) zu entziehen. Zur Ultrafiltration startet die Steuereinheit 18 die Ultrafiltrationspumpe 17.

In Abhängigkeit von den Förderraten der zweiten und dritten Pumpe 14, 15 kann frische Dialysierflüssigkeit kontinuierlich in einem relativ kurzen oder einem relativ langen Zeitraum zugeführt und das gewünschte Verhältnis zwischen frischer und verbrauchter Dialysierflüssigkeit in dem Flüssigkeitskreislauf II eingestellt werden.

An den zweiten Arbeitstakt (Fig. 2) des Arbeitszyklus schließt sich dann wieder der erste Arbeitstakt (Fig. 1) eines nachfolgenden Zyklus an.

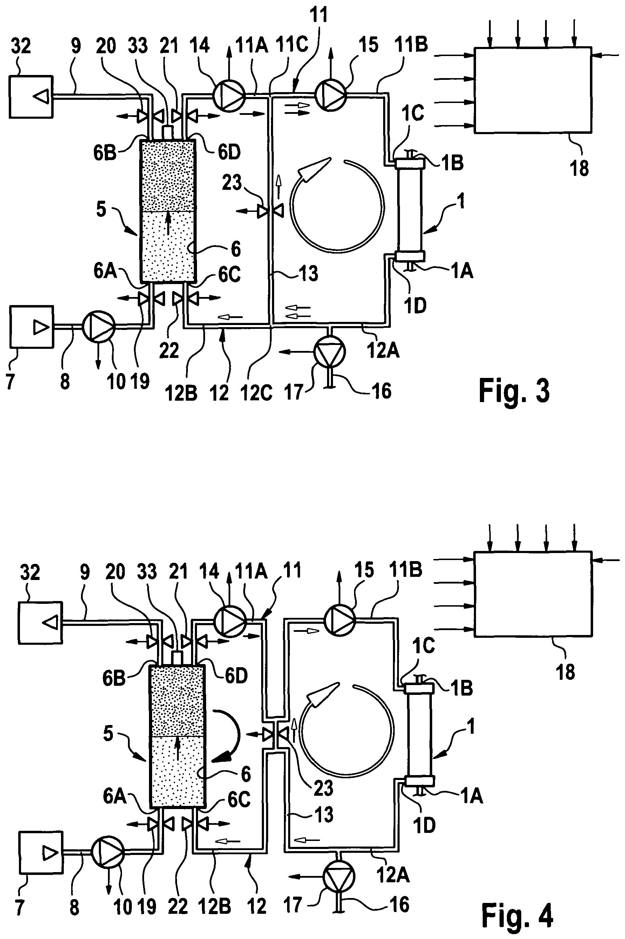

Fig. 3 zeigt ein zweites Ausführungsbeispiel der Dialysevorrichtung, das sich von der unter Bezugnahme auf die Fig. 1 und 2 beschriebenen Ausführungsform nur dadurch unterscheidet, dass in die Bypass-Leitung 13 ein fünftes Absperrorgan 23 geschaltet ist, das ebenfalls von der zentralen Steuereinheit 18 betätigt wird. Die einander entsprechenden Teile sind daher mit den gleichen Bezugszeichen versehen. Das Absperrorgan 23 in der Bypass-Leitung 13 ist während des Betriebs der Dialysevorrichtung grundsätzlich offen. Das Absperrorgan 23 kann aber auch zur Unterbrechung der Zirkulation der Dialysierflüssigkeit in dem Flüssigkeitskreislauf II geschlossen werden.
Zum Befüllen und Entlüften des Flüssigkeitssystems wird das Absperrorgan 23 in der Bypass-Leitung 13 geschlossen, so dass die Flüssigkeitsströmung durch die Bypass-Leitung unterbrochen ist. Weiterhin wird das zweite Absperrorgan 20 geschlossen, so dass Flüssigkeit nicht in den Ablauf 32 gelangen kann. Die Flüssigkeit wird über die erste Zuflussleitung 8 bei laufender Pumpe 10 der Bilanzkammer 6 zugeführt. Bei laufenden Pumpen 14 und 15 kann die Flüssigkeit durch die zweite Ablaufleitung 11, den Dialysator 1 und die zweite Zuflussleitung 12 strömen. Zur Entlüftung ist ein Entlüftungsventil 33 an der Oberseite der Bilanzkammer 6 vorgesehen, das in Fig. 3 nur deutungsweise dargestellt ist.

Fig. 4 zeigt eine alternative Ausführungsform der Dialysevorrichtung von Fig. 3. Das Ausführungsbeispiel von Fig. 4 unterscheidet sich von der Ausführungsform von Fig. 3 nur durch die Führung der zweiten Abflussleitung 11 und der zweiten Zuflussleitung 12 und die Länge der Bypass-Leitung 13. Die einander entsprechenden Teile sind wieder mit den gleichen Bezugszeichen versehen. Zur Verringerung des Totvolumens, in dem sich bei der Befüllung des Flüssigkeitssystems Luft ansammeln könnte, werden die zweite Abfluss- und Zuflussleitung 11, 12 direkt an die Anschlüsse des Absperrorgans 23 herangeführt, so dass das Volumen des Bypasses 13 auf ein Minimum reduziert werden kann.

Die erfindungsgemäße Vorrichtung zum Versorgen des Dialysators mit Dialysierflüssigkeit hat den Vorteil, dass auf eine Bilanziereinheit mit zwei Bilanzkammern verzichtet werden kann. Auch mit einer Bilanziereinheit, die nur über eine Bilanzkammer verfügt, kann eine kontinuierliche Strömung von Dialysierflüssigkeit durch den Dialysator während der Bilanzierung von frischer gegen verbrauchte Dialysierflüssigkeit aufrecht erhalten werden.

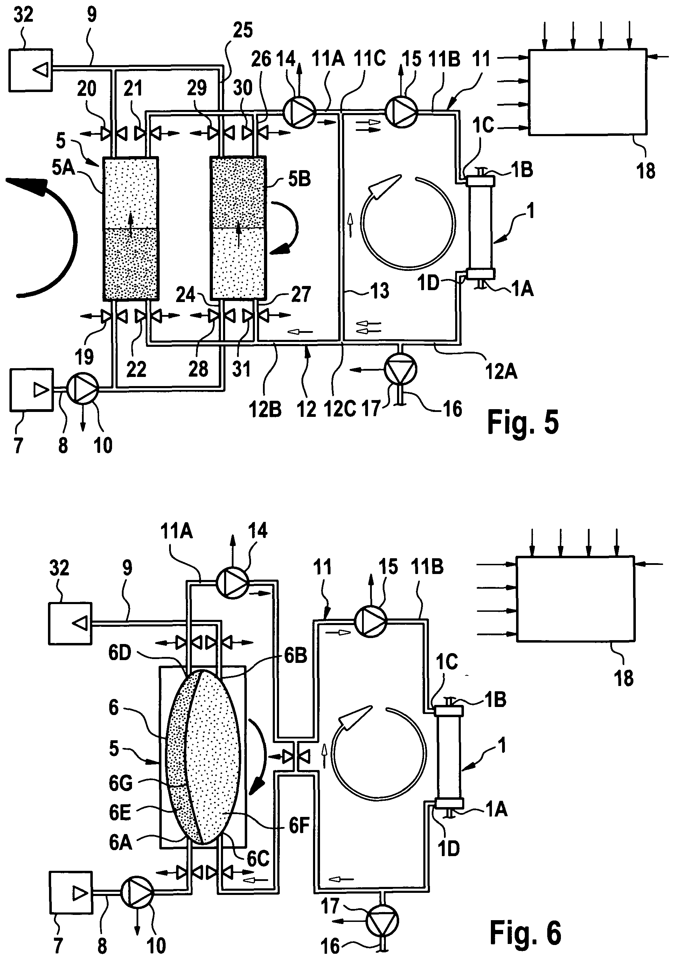

Fig. 5 zeigt der Vollständigkeit halber eine Dialysevorrichtung mit zwei parallel geschalteten Bilanzkammern 6A, 6B, die wechselweise betrieben werden. Das Ausführungsbeispiel von Fig. 5 unterscheidet sich von der Ausführungsform von Fig. 1 und Fig. 2 dadurch, dass die zweite Bilanzkammer 6B mit den zugehörigen Zu- und Ablaufleitungen 24, 25, 26, 27, in die jeweils Absperrorgane 28, 29, 30, 31 geschaltet sind, der ersten Bilanzkammer 6A parallel geschaltet ist. Die Bilanziereinheit 6 mit zwei Bilanzkammern 6A, 6B wird in bekannter Weise betrieben, wie beispielsweise in der DE 28 38 414 beschrieben ist.

Fig. 6 zeigt ein weiteres Ausführungsbeispiel, das sich von der Ausführungsform von Fig. 4 nur dadurch unterscheidet, dass die Bilanzkammer 6 durch eine flexible Trennwand 6G in eine erste Kammerhälfte 6E und eine zweite Kammerhälfte 6F unterteilt ist. Bei dieser Ausführungsform steht der erste Einlass 6A und der zweite Auslass 6D mit der ersten Kammerhälfte 6E in Verbindung, während der zweite Einlass 6C und der erste Auslass 6B mit der zweiten Kammerhälfte 6F in Verbindung steht. Beide Kammerhälften 6E und 6F der Bilanzkammer 6 werden bei dieser Ausführungsform wechselweise mit frischer und verbrauchter Dialysierflüssigkeit befüllt. Die einander entsprechenden Teile sind wieder mit den gleichen Bezugszeichen versehen. Auch bei dieser Ausführungsform erlaubt der Flüssigkeitskreislauf II die Aufrechterhaltung der Strömung von Dialysierflüssigkeit durch den Dialysator 1 während des Befüllens der Bilanzkammer 6 mit frischer Dialysierflüssigkeit. Wenn die jeweils eine Kammerhälfte 6E bzw. 6F mit frischer Dialysierflüssigkeit befüllt wird, wird die verbrauchte Dialysierflüssigkeit aus der jeweils anderen Kammerhälfte 6F bzw. 6E verdrängt.

## Patentansprüche

1. Vorrichtung zum Fördern von Flüssigkeiten in die Behandlungseinheit einer medizinische Behandlungsvorrichtung mit
einer Bilanziereinheit (5), die mindestens eine Bilanzkammer (6) aufweist,
einer zu der Bilanzkammer führenden ersten Zuflussleitung (8) zum Zuführen von Flüssigkeit aus einer Flüssigkeitsquelle (7) in die Bilanzkammer und einer von der Bilanzkammer abgehenden ersten Abflussleitung (9) zum Abführen von Flüssigkeit aus der Bilanzkammer in einen Ablauf (32),
einer von der Bilanzkammer abgehenden zweiten Abflussleitung (11) zum Abführen von Flüssigkeit aus der Bilanzkammer in eine Behandlungseinheit (1) und einer zu der Bilanzkammer führenden zweiten Zuflussleitung (12) zum Zuführen von Flüssigkeit aus der Behandlungseinheit in die Bilanzkammer,
Mitteln (10, 14, 15) zum Fördern von Flüssigkeit in die und/oder aus der Bilanzkammer und Mitteln (19, 20, 21, 22) zum Unterbrechen des Zuflusses von Flüssigkeit in die Bilanzkammer und/oder Abflusses von Flüssigkeit aus der Bilanzkammer,
einer Steuereinheit (18) zum Ansteuern der Mittel (10, 14, 15) zum Fördern von Flüssigkeit und Mittel (19, 20, 21, 22) zum Unterbrechen des Zuflusses und/oder Abflusses von Flüssigkeit,
einen die zweite Abflussleitung (11) mit der zweiten Zuflussleitung (12) verbindenden Bypass (13), der derart ausgebildet ist, dass ein die Behandlungseinheit (1) einschließender Flüssigkeitskreislauf (II) unter vollständiger oder teilweiser Umgehung der Bilanzkammer (6) geschaffen wird, wobei
die zweite Abflussleitung (11) einen in Flussrichtung ersten und einen zweiten Abschnitt (11A, 11 B) und die zweite Zuflussleitung (12) einen in Flussrichtung ersten und einen zweiten Abschnitt (12A, 12B) aufweist, und der eine Anschluss des Bypasses (13) an den Verbindungspunkt (11C) zwischen dem ersten und zweiten Abschnitt der zweiten Abflussleitung und der andere Anschluss des Bypasses an den Verbindungspunkt (12C) zwischen dem ersten und zweiten Abschnitt der zweiten Zuflussleitung angeschlossen ist, und
**dadurch gekennzeichnet, dass**
die Mittel zum Fördern von Flüssigkeit eine erste Pumpe (14), die in dem ersten Abschnitt (11A) der zweiten Abflussleitung (11) angeordnet ist, und eine zweite Pumpe (15) aufweisen, die in dem zweiten Abschnitt (11B) der zweiten Abflussleitung (11) angeordnet ist.

2. Vorrichtung nach Anspruch 1, **dadurch gekennzeichnet, dass** die Mittel (10, 14, 15) zum Fördern von Flüssigkeit und Mittel (19, 20, 21, 22) zum Unterbrechen des Zuflusses und/oder Abflusses von Flüssigkeit derart ausgebildet sind, dass eine Flüssigkeitsströmung in dem die Behandlungseinheit (1) einschließenden Flüssigkeitskreislauf (II) mit einer vorgegebenen Flussrate einstellbar ist.

3. Vorrichtung nach Anspruch 2, **dadurch gekennzeichnet, dass** die Steuereinheit (18) derart ausgebildet ist, dass
in einem ersten Arbeitstakt eines ersten Arbeitszyklus von aufeinanderfolgenden Arbeitszyklen die Bilanzkammer (6) über die erste Zuflussleitung (8) mit Flüssigkeit aus der Flüssigkeitsquelle (7) unter Verwerfung von Flüssigkeit aus der Bilanzkammer über die erste Abflussleitung (9) in den Ablauf (32) befüllt wird, wobei Flüssigkeit unter vollständiger Umgehung der Bilanzkammer in dem die Behandlungseinheit einschließenden Flüssigkeitskreislauf (II) zirkuliert, und
in einem zweiten Arbeitstakt der aufeinanderfolgenden Arbeitszyklen aus der Bilanzkammer (6) Flüssigkeit über die zweite Abflussleitung (11) abgeführt und Flüssigkeit über die zweite Zuflussleitung (12) der Bilanzkammer zugeführt wird, wobei Flüssigkeit in dem die Behandlungseinheit einschließenden Flüssigkeitskreislauf (II) zirkuliert.

4. Vorrichtung nach einem der Ansprüche 1 bis 3, **dadurch gekennzeichnet, dass** die Mittel zum Fördern von Flüssigkeit eine dritte Pumpe (10) aufweisen, die in der ersten Zuflussleitung (8) angeordnet ist.

5. Vorrichtung nach einem der Ansprüche 1 bis 4, **dadurch gekennzeichnet, dass** die Mittel zum Unterbrechen des Zuflusses und/oder Abflusses von Flüssigkeit aufweisen:
einem in der ersten Zuflussleitung (8) angeordneten ersten Absperrorgan (19),
einem in der ersten Abflussleitung (9) angeordneten zweiten Absperrorgan (20) einem in der zweiten Abflussleitung (11) angeordneten dritten Absperrorgan (21),
einem in der zweiten Zuflussleitung (12) angeordneten vierten Absperrorgan (22).

6. Vorrichtung nach einem der Ansprüche 1 bis 5, **dadurch gekennzeichnet, dass** die Mittel zum Fördern von Flüssigkeit und Mittel zum Unterbrechen des Zuflusses und/oder Abflusses von Flüssigkeit ein fünftes Absperrogan (23) aufweisen, das in dem Bypass (13) angeordnet ist.

7. Vorrichtung nach Anspruch 5 und 6, **dadurch gekennzeichnet, dass** die Steuereinheit (18) derart ausgebildet ist, dass
in einem ersten Arbeitstakt eines ersten Arbeitszyklus von aufeinanderfolgenden Arbeitszyklen das erste und zweite Absperrorgan (19, 20) geöffnet und das dritte und vierte Absperrorgan (21, 22) geschlossen sind, wobei die zweite und dritte Pumpe (15, 10) in Betrieb sind, und
in einem zweiten Arbeitstakt der aufeinanderfolgenden Arbeitszyklen das erste und zweite Absperrorgan (19, 20) geschlossen und das dritte und vierte Absperrorgan (21, 22) geöffnet sind, wobei die erste und zweite Pumpe (14, 15) in Betrieb sind.

8. Vorrichtung nach Anspruch 7, **dadurch gekennzeichnet, dass** die Steuereinheit (18) derart ausgebildet ist, dass in dem zweiten Arbeitstakt die erste und zweite Pumpe (14, 15) mit unterschiedlichen Förderarten betrieben werden.

9. Vorrichtung nach Anspruch 7 oder 8, **dadurch gekennzeichnet, dass** die Steuereinheit (18) derart ausgebildet ist, dass die zweite Pumpe (15) in dem zweiten Arbeitstakt mit einer Förderrate betrieben wird, die größer als die Förderrate der ersten Pumpe (14) ist.

10. Vorrichtung zur extrakorporalen Blutbehandlung mit einer Vorrichtung nach einem der Ansprüche 1 bis 9.

11. Extrakorporale Blutbehandlungsvorrichtung nach Anspruch 10, **dadurch gekennzeichnet, dass** die Blutbehandlungsvorrichtung eine Dialysevorrichtung ist, wobei die Blutbehandlungseinheit ein Dialysator (1) ist.

## Claims

1. A device for conveying fluid into the treatment unit of a medical treatment apparatus with
a balancing unit (5) which comprises at least one balancing chamber (6),
a first supply line (8) leading to the balancing chamber for supplying fluid from a fluid source (7) into the balancing chamber and a first discharge line (9) leading away from the balancing chamber for discharging fluid from the balancing chamber into a drain (32),
a second discharge line (11) leading away from the balancing chamber for discharging fluid from the balancing chamber into a treatment unit (1) and a second supply line (12) leading to the balancing chamber for supplying fluid from the treatment unit into the balancing chamber,
means (10, 14, 15) for conveying fluid into and/or out of the balancing chamber and means (19, 20, 21, 22) for interrupting the supply of fluid into the balancing chamber and/or discharge of fluid from the balancing chamber,
a control unit (18) for controlling the means (10, 14, 15) for conveying fluid and means (19, 20, 21, 22) for interrupting the supply and/or discharge of fluid,
a bypass (13) connecting the second discharge line (11) to the second supply line (12), which is designed such that a fluid circuit (II) including the treatment unit (1) is created thereby completely or partially bypassing the balancing chamber (6),
wherein the second discharge line (11) comprises a first and a second section (11A, 11B) in the flow direction and the second supply line (12) comprises a first and a second section (12A, 12B) in the flow direction, and one connection of the bypass (13) is connected to the junction point (11C) between the first and the second section of the second discharge line and the other connection of the bypass is connected to the junction point (12C) between the first and the second section of the second supply line, and
**characterised in that** the means for conveying fluid comprise a first pump (14), which is disposed in the first section (11A) of the second discharge line (11), and a second pump (15), which is disposed in the second section (11B) of the second discharge line (11).

2. The device according to claim 1, **characterised in that** the means (10, 14, 15) for conveying fluid and means (19, 20, 21, 22) for interrupting the supply and/or discharge of fluid are designed such that a fluid flow in the fluid circuit (II) including the treatment unit (1) can be adjusted at a predetermined flow rate.

3. The device according to claim 2, **characterised in that** the control unit (18) is designed such that,
in a first work step of a first work cycle of successive work cycles, the balancing chamber (6) is filled via the first supply line (8) with fluid from the fluid source (7), fluid thereby being displaced out of the balancing chamber via the first discharge line (9) into the drain (32), whereby fluid circulates in the fluid circuit (II) including the treatment unit completely bypassing the balancing chamber, and,
in a second work step of the successive work cycles, fluid is carried away from the balancing chamber (6) via the second discharge line (11) and fluid is fed to the balancing chamber via the second supply line (12), whereby fluid circulates in the fluid circuit (II) including the treatment unit.

4. The device according to any one of claims 1 to 3, **characterised in that** the means for conveying fluid comprise a third pump (10), which is disposed in the first supply line (8).

5. The device according to any one of claims 1 to 4, **characterised in that** the means for interrupting the supply and/or discharge of fluid comprise:
a first shut-off element (19) disposed in the first supply line (8),
a second shut-off element (20) disposed in the first discharge line (9),
a third shut-off element (21) disposed in the second discharge line (11),
a fourth shut-off element (22) disposed in the second supply line (12).

6. The device according to any one of claims 1 to 5, **characterised in that** the means for conveying fluid and means for interrupting the supply and/or discharge of fluid comprise a fifth shut-off element (23) which is disposed in the bypass (13).

7. The device according to claims 5 and 6, **characterised in that** the control unit (18) is designed such that the first and second shut-off elements (19, 20) are opened and the third and fourth shut-off elements (21, 22) are closed in a first work step of a first work cycle of successive work cycles, the second and third pumps (15, 10) being in operation, and
the first and second shut-off elements (19, 20) are closed and the third and fourth shut-off elements (21, 22) are opened in a second work step of successive work cycles, the first and second pumps (14, 15) being in operation.

8. The device according to claim 7, **characterised in that** the control unit (18) is designed such that the first and second pumps (14, 15) are operated in different delivery modes in the second work step.

9. The device according to claim 7 or 8, **characterised in that** the control unit (18) is designed such that the second pump (15) is operated in the second work step at a delivery rate which is greater than the delivery rate of the first pump (14).

10. An apparatus for extracorporeal blood treatment with a device according to any one of claims 1 to 9.

11. The extracorporeal blood treatment apparatus according to claim 10, **characterised in that** the blood treatment apparatus is a dialysis apparatus, the blood treatment unit being a dialyser (1).

## Revendications

1. Dispositif pour le transport de fluides dans l'unité de traitement d'un dispositif de traitement médical comportant
une unité d'équilibrage (5) qui présente au moins une chambre d'équilibrage (6),
une première conduite d'admission (8) conduisant à la chambre d'équilibrage pour l'acheminement de fluide d'une source de fluide (7) dans la chambre d'équilibrage et une première conduite d'évacuation (9) partant de la chambre d'équilibrage pour l'évacuation de fluide de la chambre d'équilibrage en une opération (32),
une deuxième conduite d'évacuation (11) partant de la chambre d'équilibrage pour l'évacuation de fluide de la chambre d'équilibrage dans une unité de traitement (1) et une deuxième chambre d'admission conduisant à la chambre d'équilibrage pour l'acheminement de fluide de l'unité de traitement dans la chambre d'équilibrage,
des moyens (10, 14, 15) pour le transport d'un fluide dans et/ou hors de la chambre d'équilibrage et des moyens (19, 20, 21, 22) pour interrompre l'admission de fluide dans la chambre d'équilibrage et/ou l'évacuation de fluide de la chambre d'équilibrage,
une unité de commande (18) pour commander les moyens (10, 14, 15) pour le transport d'un fluide et des moyens (19, 20, 21, 22) pour interrompre l'admission et/ou l'évacuation de fluide,
une dérivation (13) reliant la deuxième conduite d'évacuation (11) à la deuxième conduite d'admission (12), qui est conçue de telle sorte qu'un circuit de fluide (II) incluant l'unité de traitement (1) est créé en contournant complètement ou partiellement la chambre d'équilibrage (6), dans lequel
la deuxième conduite d'évacuation (11) présente un premier et un deuxième segment (11A, 11B) dans une direction d'écoulement et la deuxième conduite d'admission (12) présente un premier et un deuxième segment (12A, 12B) dans une direction d'écoulement, et l'un raccordement de la dérivation (13) est raccordé au point de liaison (11C) entre le premier et le deuxième segment de la deuxième conduite d'évacuation et l'autre raccordement de la dérivation est raccordé au point de liaison (12C) entre le premier et le deuxième segment de la deuxième conduite d'admission, et
**caractérisé en ce que**
les moyens pour le transport de fluide présentent une première pompe (14) qui est agencée dans le premier segment (11A) de la deuxième conduite d'évacuation (11) et une deuxième pompe (15) qui est agencée dans le deuxième segment (11B) de la deuxième conduite d'évacuation (11).

2. Dispositif selon la revendication 1, **caractérisé en ce que** les moyens (10, 14, 15) pour le transport de fluide et les moyens (19, 20, 21, 22) pour l'interruption de l'admission et/ou de l'évacuation de fluide sont conçus de telle sorte qu'un courant de fluide peut être réglé à un débit prédéterminé dans le circuit de fluide (II) incluant l'unité de traitement (1).

3. Dispositif selon la revendication 2, **caractérisé en ce que** l'unité de commande (18) est conçue de telle sorte que
dans un premier temps de travail d'un premier cycle de travail de cycles de travail consécutifs, la chambre d'équilibrage (6) est remplie par la première conduite d'admission (8) avec un fluide de la source de fluide (7) en rejetant du fluide de la chambre d'équilibrage par la première conduite d'évacuation (9) dans l'opération (32), dans lequel du fluide circule en contournant complètement la chambre d'équilibrage dans le circuit de fluide (II) incluant l'unité de traitement, et
dans un deuxième temps de travail des cycles de travail consécutifs, un fluide est évacué de la chambre d'équilibrage (6) par la deuxième conduite d'évacuation (11) et du liquide est acheminé par la deuxième conduite d'admission (12) de la chambre d'équilibrage, dans lequel du fluide circule dans le circuit de fluide incluant l'unité de traitement.

4. Dispositif selon l'une des revendications 1 à 3, **caractérisé en ce que** les moyens pour le transport de fluide présentent une troisième pompe (10) dans laquelle la première conduite d'admission (8) est agencée.

5. Dispositif selon l'une des revendications 1 à 4, **caractérisé en ce que** les moyens pour interrompre l'admission et/ou l'évacuation de fluide présentent :
un premier organe de blocage (19) agencé dans la première conduite d'admission (8),
un deuxième organe de blocage (20) agencé dans la première conduite d'évacuation (9),
un troisième organe de blocage (21) agencé dans la deuxième conduite d'évacuation (11),
un quatrième organe de blocage (22) agencé dans la deuxième conduite d'admission (12).

6. Dispositif selon l'une des revendications 1 à 5, **caractérisé en ce que** les moyens pour le transport de fluide et les moyens pour l'interruption de l'admission et/ou de l'évacuation de fluide présentent un cinquième organe de blocage (23) qui est agencé dans la dérivation (13).

7. Dispositif selon la revendication 5 ou 6, **caractérisé en ce que** l'unité de commande (18) est conçue de telle sorte que
dans un premier temps de travail d'un premier cycle de travail de cycles de travail consécutifs, les premier et deuxième organes de blocage (19, 20) sont ouverts et les troisième et quatrième organes de blocage (21, 22) sont fermés, dans lequel les deuxième et troisième pompes (15, 10) fonctionnent, et
dans un deuxième temps de travail des cycles de travail consécutifs, les premier et deuxième organes de blocage (19, 20) sont fermés et les troisième et quatrième organes de blocage (21, 22) sont ouverts, dans lequel les première et deuxième pompes (15, 10) sont en marche.

8. Dispositif selon la revendication 7, **caractérisé en ce que** l'unité de commande (18) est conçue de telle sorte que dans le deuxième temps de travail, les première et deuxième pompes (14, 15) sont actionnées selon différents régimes.

9. Dispositif selon la revendication 7 ou 8, **caractérisé en ce que** l'unité de commande (18) est conçue de telle sorte que la deuxième pompe (15) est actionnée dans le deuxième temps de travail à un régime qui est supérieur au régime de la première pompe (14).

10. Dispositif de traitement extracorporel du sang comportant un dispositif selon l'une des revendications 1 à 9.

11. Dispositif de traitement extracorporel du sang selon la revendication 10, **caractérisé en ce que** le dispositif de traitement du sang est un dispositif de dialyse, dans lequel l'unité de traitement du sang est un dialyseur (1).
